# EUROPEAN PATENT APPLICATION

(11) **EP 4 467 070 A1**
(43) Date of publication of application: **27.11.2024**
(21) Application number: 23174854.2
(22) Date of filing: 23.05.2023
(51) Int. Cl.: A61B 5/024, A61B 5/1455

(54) **A DEVICE AND METHOD FOR PERFORMING OXYGEN SATURATION MEASUREMENTS**

(71) Applicant: Nederlandse Organisatie voor toegepast-natuurwetenschappelijk Onderzoek TNO, 2595 DA 's-Gravenhage (NL)
(72) Inventor: AKKERMAN, Hylke Broer, 2595 DA 's-Gravenhage (NL); WEBER, Jan, 2595 DA 's-Gravenhage (NL); UZUNBAJAKAVA, Natallia Eduardauna, 2595 DA 's-Gravenhage (NL); PETRE, Adrian-Razvan, 2595 DA 's-Gravenhage (NL); KHATOUN, Ahmad, 2595 DA 's-Gravenhage (NL); SOUNDARARAJAN, Anand, 2595 DA 's-Gravenhage (NL)
(74) Representative: V.O.

(57) **Abstract**

The invention relates to a device and method for performing oxygen saturation measurements. A longitudinal channel structure is provided that is removably insertable into an ear canal, the channel structure having an outer portion configured to expand into an expanded position, wherein in the expanded position the channel structure is configured such that its outer portion exerts pressure against an inner surface of the ear canal of the user. A longitudinal open channel is formed by a body of the channel structure to maintain an opening in the ear canal when the channel structure is placed in its expanded position in the ear canal, wherein the channel structure includes a plurality of photosensitive sensors which are utilized for performing pulse oximetry measurements when the device is in the expanded position.

## Description

### FIELD OF THE INVENTION

The invention relates to a device for performing oxygen saturation measurements. The invention also relates to a method for performing oxygen saturation measurements. Furthermore, the invention relates to a method of deploying the device. The invention also relates to a method of using the device. The invention also relates to devices and methods for non-invasive monitoring of vital signs in the ear-canal, with a particular focus on oxygen saturation measurements.

### BACKGROUND TO THE INVENTION

In recent years, pulse oximetry has become a critical tool for monitoring patients' oxygen saturation levels. This non-invasive method measures the percentage of hemoglobin that is saturated with oxygen in the blood. Traditional pulse oximetry devices are typically worn on the fingertip or earlobe, which can cause discomfort, irritation, or even dislocation during extended periods of use. Moreover, these conventional devices may not provide accurate measurements in certain situations, such as low blood flow or in cases where the user has cold extremities.

In addition to these issues, existing pulse oximetry devices often suffer from poor fit, inadequate fixation, or interference from ambient light. These factors can lead to inaccurate measurements or the need for repeated adjustments. Furthermore, conventional devices can obstruct the ear canal when used for oxygen saturation measurements, making it difficult for users to hear their surroundings or communicate effectively.

As a result, there is a growing need for an improved pulse oximetry device that provides a secure and comfortable fit, ensures accurate and reliable measurements, and allows for convenience for the user during use. This would greatly benefit users in various settings, including medical facilities, sport environments, personal wellness, remote patient monitoring setting, home care environments, and various other situations where continuous monitoring of oxygen saturation levels may be beneficial or necessary.

There is a strong desire for a user-friendly, comfortable, and non-invasive design of a device that can enable monitoring of an individual's physiological parameters, such as oxygen levels in the blood, heart rate, heart rate variability, and respiration rate, in an efficient and accurate way.

### SUMMARY OF THE INVENTION

It is an object of the invention to provide for a method and a system that obviates at least one of the above mentioned drawbacks.

Additionally or alternatively, it is an object of the invention to improve the monitoring of an individual's physiological parameters, such as oxygen levels in the blood.

Additionally or alternatively, it is an object of the invention to provide for an improved ear-canal device for performing oxygen saturation measurements.

Thereto, the invention provides for a device for performing oxygen saturation measurements, the device comprising a longitudinal channel structure removably insertable into an ear canal, the channel structure having an outer portion configured to expand into an expanded position, wherein in the expanded position the channel structure is configured such that its outer portion exerts pressure against an inner surface of the ear canal of the user, wherein a longitudinal open channel is formed by a body of the channel structure to maintain an opening in the ear canal when the channel structure is placed in its expanded position in the ear canal, wherein the channel structure includes a plurality of photosensitive sensors which are utilized for performing pulse oximetry measurements when the device is in the expanded position.

A convenient and non-invasive method for measuring oxygen saturation is provided by the device. The expandable channel structure ensures a secure fit in the ear canal. The device maintains an opening in the ear canal for user comfort and safety. The longitudinal channel structure allows for easy insertion into the ear canal. The outer portion expands to create pressure against the inner surface of the ear canal, ensuring the secure fit resulting in accurate measurements by the photosensitive sensors. The photosensitive sensors can be configured in the channel structure such as to enable pulse oximetry measurements to be carried out when the device is inserted and installed in the ear-canal of the user.

Advantageously, the device is able to resist movement in the ear canal/channel. In this way, the sensors of the device may pick up less motion artifacts. Motion artifacts can be caused by body movement or internal physiological factors. However, the ear canal is less susceptible to these issues, especially with proper pressure from inside out against the wall of the ear canal. The device can maintain a continuous physical contact with the wall of the ear canal in an advantageous way.

In some examples, the device is a wearable earpiece. For example, the device may be used by athletes to monitor their oxygen saturation during exercise, or may be used in hospitals for continuous oxygen saturation monitoring of patients, etc.

Optionally, the channel structure is a predominantly elongated open channel structure, wherein the open channel of the channel structure has a cross-sectional area ratio, defined as the open channel cross-sectional area divided by the total cross-sectional area of the channel structure, that is higher than 50%, more preferably higher than 60%, even more preferably higher than 70%.

A greater cross-sectional area ratio of the open channel can ensure better comfort for the user. Furthermore, it allows for easier airflow and sound passage within the ear canal. This enables the device being worn comfortably during extended periods.

The channel structure may have a highly open design. The channel structure may have a relatively large cross-sectional opening area in its longitudinal direction compared to the thickness of its outer walls in the expanded position.

Optionally, the channel structure is connected to an outer piece, wherein the outer piece extends outwardly from the user's ear when the channel structure is inserted into the ear canal, wherein the outer piece is configured to conform to the shape of the user's ear for providing a secure and comfortable fit of the outer piece of the device during use, wherein the outer piece preserves an open pathway to the channel structure, and wherein the outer piece houses at least a power supply unit and one or more further electronic components of the device.

The outer piece conforms to the user's ear for a secure and comfortable fit. The outer piece can house power supply and electronic components, keeping the part of the device that is inserted in the ear-canal compact. In this way, an open pathway can be maintained to inside of the ear. In some examples, the device has a customizable earpiece with an outer piece that adapts to the user's ear shape, providing a secure fit for long-term use.

The outer piece may be configured to ensure free flow through the channel structure. Hence, the outer piece may be configured to enable an unimpeded interface with the channel structure. Such unrestricted connectivity to the channel structure may result in an open pathway to the channel structure.

In some examples, the outer piece houses the power supply, the processing unit, and optionally a communication unit. The power supply may provide power to the electronic components such as the sensors and processing unit. The processing unit may be configured to receive data from the plurality of photosensitive sensors, process the data, and determine oxygen saturation levels in the blood based on the data. The communication unit may be configured for wireless transmission of the oxygen saturation levels and other data to an external device. The power supply may include a battery compartment, the battery compartment being configured to receive one or more replaceable or rechargeable batteries to supply power to the device.

The outer piece and/or an external device in data communication with the device may include a user interface, the user interface being configured to receive user input for controlling the device, such as turning the device on or off, adjusting settings, and/or initiating data transmission to the external device or another remote device.

Optionally, the device includes one or more light emitting units configured to transmit light at one or more wavelengths, wherein the plurality of photosensitive sensors on the channel structure are configured to receive the emitted light passing surrounding blood vessels and/or bodily tissue of the user so as to measure oxygen saturation levels in the blood of the user, wherein the one or more light emitting units are arranged on the channel structure and/or at one or more locations of the outer piece.

The plurality of light-emitting units, such as LEDs, may be configured to emit light in a pulsatile manner, the pulsatile emission being synchronized with the acquisition of data by the plurality of photosensitive sensors to minimize interference from ambient light.

Optionally, the light-emitting units and the plurality of photosensitive sensors are arranged on the body in a predetermined pattern, the pattern facilitating the efficient transmission of light from the light-emitting units to the photosensitive sensors through the surrounding tissue.

The light emitting units and photosensitive sensors can work together for accurate oxygen saturation/concentration measurements. The device can obtain measurements from surrounding blood vessels and bodily tissue. The light emitting units can transmit light at specific wavelengths for measurement purposes, wherein the photosensitive sensors on the channel structure can be configured to receive emitted light and measure oxygen saturation levels based on the received light. For example, the device may employ light emitting units operating at 660 nm (red) and 940 nm (infrared) wavelengths to accurately measure oxygen saturation in the blood. However, other wavelengths can also be employed.

The device may include a processing unit or control unit for the adjustment of light intensity, wavelength, or pulsatile emission patterns to optimize oxygen level measurements in the blood. The processing unit may be configured to apply a calibration curve or algorithm to the received light data and emitted light data to account for variations in tissue properties, light absorption, or other factors that may affect the determination of oxygen levels in the blood.

Optionally, the outer piece is a rod shaped structure, wherein the rod shaped structure includes a protruding portion extending away from a body connected to the longitudinal channel structure.

The rod-shaped structure of the outer piece can provide additional support for the device. In some examples, the rod-shaped structure forms a stem that is shaped such that the device is comfortably held at the ear of the user. The protruding portion extends away from the body, allowing for easy attachment and removal. The outer piece with a rod-shaped structure and a protruding portion provides for easy handling. The device can be attached and detached with ease.

Optionally the rod shaped structure has a generally cylindrical shape. The rod shaped structure may be insertable in a charging device for wireless charging of a battery housed therein.

Optionally, the outer piece includes a hook-shaped attachment member configured to engage and secure the device to a user's ear by wrapping around the outer contour of the user's ear, wherein one or more light emitting units are arranged on the hook-shaped attachment member.

The hook-shaped attachment member can ensure a secure fit on the user's ear. The device can thus operate and be firmly held in place even during physical activity. Light emitting units on the attachment member provide additional measurement locations.

In some examples, the hook-shaped attachment member includes a flexible and adjustable connecting portion to enable the device to accommodate various ear sizes and shapes.

Optionally, the outer piece is configured to at least partially contact the skin around the ear when used by the user, the outer piece having a plurality of light-emitting units arranged thereon, the light-emitting units being configured to transmit light through the skin and surrounding tissue towards the photosensitive sensors on the channel structure inside the ear.

Optionally, the outer piece is connected to the channel structure by a flexible connector, the flexible connector allowing for movement and adjustment of the outer portion relative to the body to accommodate different ear and head shapes and sizes, and to ensure optimal positioning of the light-emitting units on the skin.

Optionally, the device further includes a separated unit which is physically detached from the rest of the device encompassing the channel structure and the outer piece, wherein the separated unit is configured to be placeable at or in proximity of the ear, and wherein the separated unit includes one or more light emitting units, and wherein the device includes a wireless interface between the separated unit and said rest of the device, wherein the wireless interface is configured to enable data communication over one or more wireless protocols for synchronized operation.

The separated unit provides flexibility in device placement and can provide additional measurement capabilities. A wireless interface allows for synchronized operation and data communication between the separated unit and the rest of the device. For example, a device may be employed with a separate light emitting unit that can be placed near the ear, wirelessly (e.g. Bluetooth or similar) communicating with the main device for synchronized oxygen saturation measurements.

Optionally, the plurality of photosensitive sensors are distributed over the outer portion of the body, such that in the expanded position, the sensors are distributed over the ear wall of the ear canal, wherein the distributed photosensitive sensors are embedded in a predetermined substantially uniform pattern on the surface of the body, wherein the plurality of photosensitive sensors are disposed in multiple rows and columns on the surface of the body, allowing for the collection of measurement data from various locations on the inner surface of the ear canal.

The distributed photosensitive sensors allow for more accurate and comprehensive measurements. The predetermined uniform pattern ensures consistent data collection on the inner surface of the ear canal. In some examples, the device includes photosensitive sensors arranged in multiple rows and columns, providing measurement data from different locations within the ear canal for improved accuracy.

Optionally, the plurality of photosensitive sensors is disposed along the perimeter of the body, providing a continuous ring of sensors in contact with the ear wall.

Optionally, the plurality of photosensitive sensors is arranged in a concentric configuration on the outer portion of the body of the channel structure. The concentric configuration can enable measurement of oxygen levels at various depths within the ear wall tissue.

Optionally, the plurality of photosensitive sensors is arranged in a spiral or helical pattern on the surface of the body, the spiral or helical pattern providing comprehensive coverage of the ear wall and facilitating more accurate oxygen measurements.

Optionally, the plurality of photosensitive sensors is selectively activated based on their contact quality with the ear wall, ensuring that only sensors with optimal contact contribute to the oxygen measurement data.

Optionally, the device includes a processing unit, wherein the processing unit is configured to selectively use data from the plurality of photosensitive sensors based on the received light data such that only sensors with adequate data contribute to the oxygen measurement.

The processing unit can selectively use data from photosensitive sensors, improving measurement accuracy. In this way, advantageously, it can be ensured that only sensors with adequate data contribute to the oxygen measurement. The accuracy of oxygen saturation measurements can be significantly enhanced by using only reliable sensor data.

Light can be received by the plurality of photosensitive sensors from different angles. The device may be configured to select the best-aligned photosensitive sensors. Each photosensitive sensor in an array, pattern or matrix of photosensitive sensors may not produce the same signal quality for every light emitting unit, which is attributable to both the design of the device as well as physiological factors (e.g., veins in the body). By comparing the signals of the plurality of photosensitive sensors, it can be identified where the best signal is obtained.

The light detected by the photosensitive sensors may include a DC component (e.g. affected by factors like pigment, skin type, and other physiological characteristics) and an AC component (smaller than the DC component, resulting from pulsation). Different measurements can be taken depending on what is being observed, such as peak-to-peak AC component, DC level, and AC/DC ratio.

Advantageously, if the photosensitive sensors are arranged circumferentially on the channel structure and light sources are placed on the exterior, it is possible to determine which photodetectors provide the best signal for a specific interest, without knowing the structure's orientation. The best signal may depend on the desired measurement, with most applications focusing on the AC signal's (relative) strength.

The pattern of photosensitive sensors on the outer portion of the channel structure can enable identifying information that traditional PPG devices cannot detect. In certain positions, a light band may pass through a vein, producing a stronger pulse on a specific photodiode where it was not initially anticipated. Various factors can be analyzed to determine which photodiode(s) should be used for measurements.

Optionally, the channel structure is configured to be collapsible for insertion into the ear canal and expandable to provide fixation within the ear canal, wherein the channel structure includes a flexible arrangement of a mesh-type structure, wherein the channel structure has a first configuration that is radially contracted, and a second configuration that is radially expanded.

The collapsible channel structure allows for easy insertion and removal. Furthermore, the expandable design can provide a secure fit within the ear canal. The channel structure can be easily adjustable between radially contracted and expanded configurations.

In some examples, the mesh-like structure of the device can be manufactured using a variety of configurations, including but not limited to a braided configuration, an open tubing configuration presenting either an open or closed cell architecture, or an open or closed cell sheet formation adapted into a tubular design. Other configurations are also possible. These different structural designs can be used individually or in combination for a more complex design - for instance, a hybrid configuration using both open and closed cell designs.

It is to be understood that the selection of structure, materials, and manufacturing methods is not restricted to the aforementioned examples. Rather, any suitable techniques and materials, as are commonly employed in the manufacture of similar devices (e.g. stent-like configuration) could be adopted for the design and fabrication of the said ear device. This disclosure contemplates the use of these alternatives and does not limit the invention to a particular configuration.

The device may include an arrangement for adjusting the channel structure from said first configuration towards the second configuration, and/or vice versa.

Optionally, the channel structure includes a flexible arrangement of braided filaments, wherein in the first configuration, the channel structure is radially contracted and axially lengthened, and in the second configuration, the channel structure is radially expanded and axially shortened.

In some examples, the device includes a channel structure made of braided filaments that can be easily collapsed for insertion and expanded for a secure fit within the ear canal. Flexible arrangement of braided filaments enables the channel structure to change configurations.

In addition to the braided channel structures, the present invention also contemplates the employment of channel structures fabricated by various other means. For example, a laser cutting technique may be utilized, whereby a precision laser is used to incise intricate designs into the material without the need for a physical tool that contacts the material. This method often results in highly accurate and clean cuts, allowing for intricate channel structures.

Stamped channel structures are another potential design wherein the device is manufactured through a stamping or pressing process, which typically involves placing the material into a custom-designed die and then using a mechanical or hydraulic press to shape the material into the desired channel structure.

Moreover, the device could utilize welded channel structures. This method involves the joining of materials, typically metals or thermoplastics, by applying heat, with or without pressure, and possibly with the addition of a filler material. The advantage of this method lies in its ability to create strong, permanent bonds that withstand rigorous use.

In some embodiments, the invention can also encompass channel structures that do not undergo significant dimensional changes in their length. This means that these channel structures maintain their original length during the expansion process, which could be beneficial for certain applications where maintaining the original dimensions of the device is critical. This could include cases where precise sizing is required to ensure the appropriate fit and performance within the ear canal.

Optionally, the outer portion of the channel structure is formed by a plurality of intersecting elongate members, wherein the channel structure is configured to be adjustable from a reduced diameter for deployment into the ear canal to an expended diameter, upon application of a radially outward force from the interior of the channel structure, causing deformation of at least a subset of the elongate members to retain the expanded diameter, enabling a securing position of the channel structure within the ear canal.

The adjustable diameter can ensure a secure fit in various ear canal sizes. The expandable diameter provides better contact with the ear canal for enabling accurate measurements.

The channel structure's diameter can be adjusted for deployment and securing within the ear canal. The radially outward force from the interior of the channel structure causes deformation of elongate members to retain the expanded diameter.

In some examples, the open channel of the channel structure has a cross-sectional area ratio, defined as the open channel cross-sectional area divided by the total cross-sectional area of the channel structure, that is higher than 80%, even in some examples higher than 90%. For this purpose, the channel structure may have a stent-like design, enabling the structure to be extremely thin whilst offering structural stability. The relatively large openness can improve the perception of sound, and can also reduce the risk of irritation.

Optionally, the outer portion of the channel structure has a wall thickness smaller than 300 micron, preferably smaller than 200 micron, even more preferably smaller than 150 micron.

In some examples, the channel structure is made out of metal, that can improve the structural stability and provide for a very thin yet durable structures. However, other materials, such as plastics may also be employed.

Optionally, the channel structure comprises a flexible material, the flexible material facilitating the expansion of the outer portion of the body.

The flexible material can ensure a comfortable fit. This can facilitate expansion of the outer portion of the body for a secure fit in the ear canal. The channel structure made of flexible material allows for easy expansion and adaptation to the user's ear canal. A comfortable fit can be provided by the flexible material in an advantageous way.

In some examples, the device includes a channel structure made of silicone, allowing for a comfortable and secure fit within the ear canal.

Optionally, the channel structure is a C-shaped structure, a spiral structure, a closed or open tube structure, a flexible foam structure, or a combination of such structures.

Various expandable ear-insertable structures may be employed. The structural designs may be configured to provide options for different user preferences and needs. Each channel structure type offers unique benefits in terms of fit, comfort, and functionality. For example, a device with a C-shaped channel structure allows for easy insertion and removal. A device with a spiral structure that adapts to the user's ear canal shape can provide a more secure fit.

The C-shaped structure may be configured to partially encircle the walls of the ear cavity when the device is in the expanded position. The C-shaped structure may include a first end and a second end, the first and second ends being configured to move toward or away from each other during expansion or contraction of the body. The plurality of photosensitive sensors may be arranged along a surface of the C-shaped structure, the surface being in contact with the ear walls when the device is in the expanded position.

The C-shaped structure may be formed from a flexible and resilient material, the material allowing the body to expand and contract between the expanded position and a contracted position.

The C-shaped structure may be configured to allow for the passage of air and sound through the ear cavity when the device is in the expanded position, the open channel being substantially unobstructed by the C-shaped structure.

The spiral-shaped structure may be configured to conform to the contours of the ear cavity when the device is in the expanded position. The spiral-shaped structure may comprise a plurality of interconnected turns, the interconnected turns allowing the body to expand and contract between the expanded position and a contracted position. The plurality of photosensitive sensors may be arranged along a surface of the spiral-shaped structure, the surface being in contact with the ear walls when the device is in the expanded position. In some examples, the spiral-shaped structure is formed from a flexible and resilient material, the material allowing the body to expand and contract between the expanded position and a contracted position.

The foam material may be configured to expand and push the outer surface against the ear wall when the device is in the expanded position. The open channel can be formed in a central portion of the foam material, the open channel allowing for the passage of air and sound through the ear cavity when the device is in the expanded position. In some examples, the foam material comprises a memory/bias foam, the memory/bias foam being configured to return to its original shape after being compressed and released. The plurality of photosensitive sensors may be embedded within the foam material to contact the ear walls when the channel structure is in the expanded position.

Various alternative embodiments are envisaged for the channel structure that can be inserted into the ear and expand to make contact with the ear wall. For example, the channel structure may be a collapsible mesh structure, an adjustable band structure, a flexible tube structure, etc.

Optionally, the plurality of photosensitive sensors is configured to detect photoplethysmographic (PPG) signals at at least two different wavelengths, enabling the simultaneous measurement of oxygen saturation, heart rate, heart rate variability, and respiration rate. The oxygen saturation may be determined from the PPG signals at the two different wavelengths. The device can thus measure multiple vital signs simultaneously, providing a comprehensive understanding of an individual's physiological state.

Optionally, the open channel allowing for the passage of air and sound through the ear cavity when the device is in the expanded position.

Optionally, he outer portion forms an outer wall with a thickness smaller than 2 millimeters, preferably smaller than 1 millimeter.

Optionally, the channel structure forms an integrated structure comprising both the plurality of light-emitting units and the plurality of photosensitive sensors.

Optionally, the device includes and/or is interoperable with an actuator configured to selectively expand or contract the outer portion between the expanded position and the contracted position, wherein the outer portion of the body does not push against the walls of the ear cavity in the contracted position. The actuator may include comprises a mechanical, electrical, and/or magnetic mechanism configured to control the expansion or contraction of the channel structure.

Optionally, a processor is configured to receive data from the plurality of photosensitive sensors and determine oxygen saturation levels in the blood based on the data.

Optionally, a communication module is provided that is configured to transmit/receive data (e.g. send the oxygen saturation levels, receive control data, etc.), to an external device. The communication module may be configured for wireless transmission/reception of the data to/from the external device.

Optionally, an actuation mechanism may be provided, the actuation mechanism being configured to facilitate the transition of the channel structure from the unexpanded position to the expanded position. The actuation mechanism may comprise a shape-memory element, a mechanical expandable structure, or an inflatable structure.

Optionally, the channel structure includes a shape-memory material, the shape-memory material being capable of transitioning between the unexpanded position and the expanded position in response to stimulus.

According to an aspect, the invention provides for a method of deploying a device for performing oxygen saturation measurements, the device comprising a longitudinal channel structure removably insertable into an ear canal, the channel structure having an outer portion configured to expand in an expanded position, wherein in the expanded position the channel structure is configured such that its outer portion exerts pressure against an inner surface of the ear canal of the user, wherein a longitudinal open channel is formed by a body of the channel structure to maintain an opening in the ear canal when the channel structure is placed in its expanded position in the ear canal, wherein the channel structure includes a plurality of photosensitive sensors which are utilized for performing pulse oximetry measurements when the device is in the expanded position, wherein the method includes the steps of: positioning the channel structure into an ear canal of a user; and applying a stimulus to the channel structure in order to transition the channel structure from an unexpanded position to an expanded position such that its outer portion engages the inner surface of the ear canal.

The invention enables ear-based measurements of one or more vital signs using an open-structured design. The device may have an ear-insertable part and one or more external parts that remain at least partially outside of the ear cavity, the external part housing necessary components for the operation of the device (such as power supply, processing unit and/or other electronic components). Various designs are possible, including separated units for example wirelessly connected or one integrated unit (e.g. wiredly connected). The device may have various shapes. Preferably, a passage through the device is enabled, such that air and sound can travel towards the ear cavity. In some alternative embodiments, the device covers the ear opening, and sound is provided to the ears via a microphone arranged on the external part of the device.

The device may be inserted into the ear cavity in a contracted position. Then the channel structure may be expanded such that the outer portion of the body is adjusted to the expanded position, wherein the outer surface pushes against the walls of the ear cavity. Then, light from the plurality of photosensitive sensors may be transmitted into body tissue and/or blood vessels near the ear. Light that has passed through blood vessels can be received by the plurality of photosensitive sensors. Measured data can be processed to determine oxygen saturation levels in the blood. Optionally, the oxygen saturation levels can be transmitted to an external device and/or the cloud.

The device may have various alternative light transmission systems comprising the one or more light emitting units. In some examples, a series of LEDs are built into an arm section of a sport-like ear plug designed to fit around the ear. The human cranial bone's optical properties are well known and quite transparent. This LED/detector configuration enables oxygen level measurement from within the brain in an effective way. Such measurements can be performed in a rather easy way using the device according to the disclosure, whilst providing very valuable measurements of the vital signs of the person.

In some examples, a power supply for at least the electronics associated with the channel structure, and electronic components of the device such as processing unit, communication module, or any other electronic components are positioned outside the ear. This can be done by arranging such components in a housing that extends outside the ear. The multiple photosensitive sensors (e.g. array of photodetectors) of the channel structure are positionable inside the ear canal. The photosensitive sensors may for instance be arranged on an outer surface of the channel structure. Furthermore, the device may have a light transmission system including one or more light emitting units (e.g. LEDs). Such a light transmission system may be provided also outside the ear cavity (e.g. above and/or behind the ear), and/or it may be integrated with the channel structure (hence also located inside the ear canal on the surface of the channel structure). Various alternative embodiments are envisaged.

Optionally, the stimulus applied to the device comprises applying a mechanical force/pressure, releasing a radially compression pressure on the outer portion of the channel structure, activating a movement mechanism, changing a temperature or applying an electrical current for actuation.

Optionally, the channel structure of the device is a stent-like structure and wherein the expansion mechanism involves using a delivery device, wherein the delivery device is used for performing one or more of the following actions: inflating a balloon to expand a balloon-expandable channel structure, retracting a sheath to allow a self-expanding channel structure to expand, expanding a smaller channel structure within a larger channel structure, or mechanically expanding the channel structure by performing a mechanical movement.

The delivery device or deployment tool may be configured to hold the stent-like structure in the contracted position during insertion into the ear cavity, the deployment tool being configured to release the stent-like structure upon reaching a desired position within the ear cavity, allowing a self-expanding mechanism to expand the body to the expanded position.

The body of the channel structure may have been formed from a plurality of interconnected struts, the interconnected struts being capable of expanding and contracting to transition the body between an unexpanded position and an expanded position, wherein the outer portion of the body is configured to push against the ear walls when in the expanded position.

The interconnected struts may form a substantially cylindrical shape when in the expanded position, the cylindrical shape conforming to the contour of the ear cavity.

The stent-like structure may have a plurality of expansion rings interconnected by a plurality of longitudinal struts, the expansion rings being capable of radially expanding and contracting to adjust the size of the body when transitioning between the unexpanded and expanded positions.

The expansion rings may be formed from a shape-memory material, the shape-memory material being capable of transitioning between the unexpanded and expanded positions in response to a change in temperature or the application of an external stimulus.

The stent-like structure may comprise a helical or spiral arrangement of interconnected struts, the helical or spiral arrangement facilitating the radial expansion and contraction of the body when transitioning between the unexpanded and expanded positions.

Optionally, the stent-like structure includes a plurality of radial opening, wherein the plurality of photosensitive sensors is arranged at the plurality of radial openings. In some examples, also the light emitting diodes for emitting light are arranged at a subset of the radial openings.

In some examples, the device comprises two physically disconnected bodies, a first body comprising the plurality of light-emitting units, and a second body comprising the plurality of photosensitive sensors, the first body being configured such that the light-emitting units are arranged to contact the skin approximate or around the ear, and the second body having the photosensitive sensors that are pushed against the interior ear wall when the second body is put in the expanded position.

In some examples, the separated unit comprises a part that conforms to the shape of the top of the ear and extends towards the back of the ear when used by the user, the plurality of light-emitting units being arranged on the outer portion to emit light into the surrounding tissue.

In some examples, the separated unit and the rest of the device are communicatively coupled during operation, the communication between the two bodies being facilitated by wired or wireless communication technologies, such as Bluetooth, Wi-Fi, or near-field communication (NFC).

In some examples the device further comprises a processing unit, the processing unit being configured to receive and process data from the plurality of photosensitive sensors, and to control the operation of the plurality of light-emitting units of the first body, the processing unit being housed within one of the outer piece or separated unit, or being provided as a further separate external unit communicatively coupled to the device.

According to an aspect, the invention provides for a method for removing the ear-based device according to the disclosure from the ear cavity, the method comprising the steps of: applying a stimulus to the device to transition the body from the expanded position to the unexpanded position; grasping the device, such as by using an external tool or fingers; and gently pulling the device out of the ear cavity while it remains in the unexpanded position. The stimulus applied may be an external force for example.

It will be appreciated that different configurations of the device are envisaged, such as a single integrated structure with both light-emitting units and photosensitive sensors, or a separated design comprising two physically disconnected bodies, with one body housing the light-emitting units and the other housing the photosensitive sensors. In the latter configuration, the bodies are communicatively coupled during operation.

It will be appreciated that the channel structure can have various shapes and forms, wherein it elongated and has a hollow shape that can facilitate the flow of liquids or other materials.

It will be appreciated that the photosensitive sensors can be configured to receive light at one or more wavelengths, the light being absorbed by blood vessels in the ear walls to measure oxygen saturation levels in the blood.

It will be appreciated that any of the aspects, features and options described in view of the device apply equally to the system and the described methods. It will also be clear that any one or more of the above aspects, features and options can be combined.

### BRIEF DESCRIPTION OF THE DRAWING

The invention will further be elucidated on the basis of exemplary embodiments which are represented in a drawing. The exemplary embodiments are given by way of non-limitative illustration. It is noted that the figures are only schematic representations of embodiments of the invention that are given by way of non-limiting example.

In the drawing:
Fig. 1 shows a schematic diagram of an embodiment of a device;
Fig. 2 shows a schematic diagram of an embodiment of a device;
Fig. 3 shows a schematic diagram of an embodiment of a device;
Fig. 4 shows a schematic diagram of an embodiment of a device;
Fig. 5 shows a schematic diagram of an embodiment of a device;
Fig. 6 shows a schematic diagram of an embodiment of a device;
Fig. 7 shows a schematic diagram of an embodiment of a device; and
Fig. 8 shows a schematic diagram of a method for a channel structure.

### DETAILED DESCRIPTION

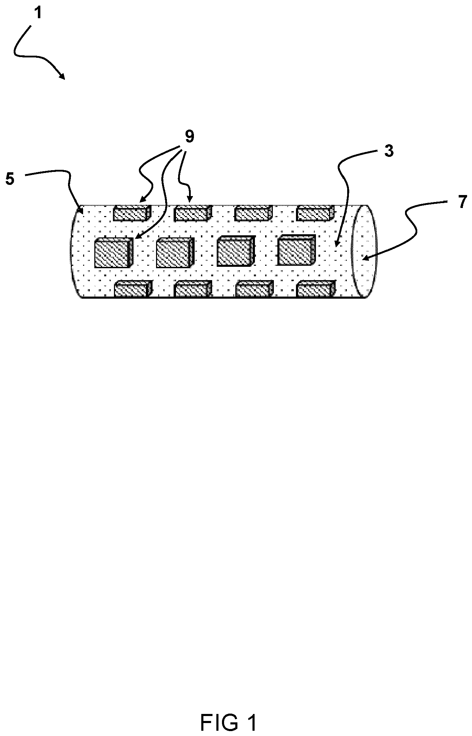

Fig. 1 shows a schematic diagram of a side view of an embodiment of a device 1 for performing oxygen saturation measurements. The device 1 comprises a longitudinal channel structure 3 removably insertable into an ear canal of a user (not shown). The channel structure 3 having an outer portion 5 configured to expand into an expanded position, wherein in the expanded position the channel structure 3 is configured such that its outer portion 5 exerts pressure against an inner surface of the ear canal of the user. The channel structure can be suitably designed and dimensioned for this purpose. A longitudinal open channel 3 is formed by a body of the channel structure 3 to maintain an opening 7 in the ear canal when the channel structure 3 is placed in its expanded position in the ear canal, wherein the channel structure 3 includes a plurality of photosensitive sensors 9 which are utilized for performing pulse oximetry measurements when the device 1 is in the expanded position.

In this example, the plurality of photosensitive sensors 9 are distributed over the outer portion 5 of the channel structure, such that in the expanded position, the photosensitive sensors 9 are distributed over the ear wall.

The device 1 may include a plurality of light-emitting units, such as LEDs, distributed over the outer portion of the channel structure, and/or provided approximate the ear for example on the outer piece of the device and/or an external device, in order to transmit light into the surrounding tissue of the ear wall or approximate the ear such that the light can be received by the photosensitive sensors.

The device 1 may be inserting into the ear canal without knowing its (rotational) orientation. The measurements carried out by the photosensitive sensors 9 (e.g., DC signals) may be employed to determine the side of the channel structure with photosensitive sensors that face the one or more light emitting units (not shown), and the side facing the opposite direction. This allows for effective selection of the photosensitive sensors 9 that are used for collecting the data for determining oxygen saturation.

In some examples, the distributed photosensitive sensors 9 may be configured to provide a spatially resolved measurement of oxygen levels in the blood within the surrounding tissue of the ear wall.

In some examples, the distributed photosensitive sensors 9 are configured to operate independently or in combination with one another, allowing for the selective activation or deactivation of individual sensors 9 based on the specific requirements of a measurement.

The processing unit of the device 1 may be configured to receive and analyze data from the distributed photosensitive sensors 9, the processing unit being adapted to determine the oxygen level in the blood of the surrounding tissue by aggregating, averaging, and/or otherwise processing the data received from the photosensitive sensors.

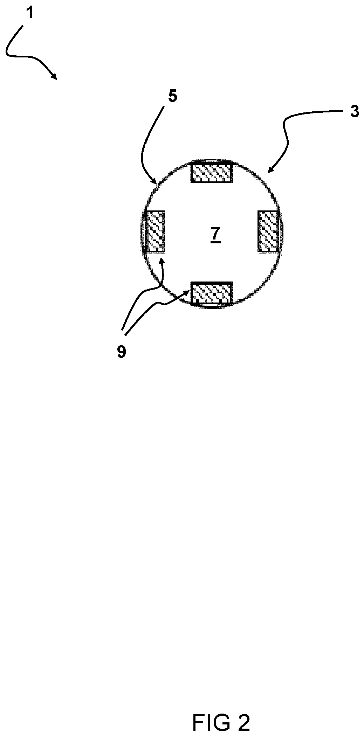

Fig. 2 shows a schematic diagram of a cross sectional view of an embodiment of a device 1. The tubular channel structure 3 can effectively avoid obstructing the ear canal, eliminating the adverse effects that are associated with such obstruction. Furthermore, the optical path of the light emitted by the light emitting units can be more advantageous by the design of the device.

Advantageously, the device 1 can determine an actual light path length between the one or more light emitting units and the photosensitive sensors, which can result in optimal signal quality by determining the orientation of the tubular channel structure 3 relative to the ear canal and one or more light emitting units. The photosensitive sensors 9 may be arranged on all sides of the channel structure to ensure the highest signal quality can be obtained regardless of its orientation, often corresponding to the shortest distance between one or more light emitting units and the photosensitive sensor 9. Moreover, by combining spatially separated light emitting units (e.g. LEDs for transmission of light), information from different light paths between the various light emitting unit and photos-sensitive sensor combinations can be determined, possibly revealing different biomedical signals due to local physiological differences.

In this example, the channel structure 3 is a predominantly elongated open channel structure, wherein the open channel 7 of the channel structure 3 has a cross-sectional area ratio, defined as the open channel cross-sectional area divided by the total cross-sectional area of the channel structure 3, that is higher than 90%.

A high cross-sectional area ratio can be obtained by for example using a self-expanding (metallic) frame (stent) channel structure. This effectively allows the ear-canal PPG device 1 to be very thin, thus enabling most of the ear canal to remain open. This feature is especially useful when used in combination with an ear microphone. The weight of such a system is also much lower than existing foam-based systems, making it more resistant to movement. As a result, it could be used during sports activities. The combination of external light emitting units (e.g. LEDs) and internally placed photosensitive sensors is also advantageous, as a light path through the skull can be used for performing the measurements. However, it is also envisaged that the light emitting units and the photosensitive sensors are arranged on the same channel structure. Various distributions and patterns of the arrangement of the photosensitive sensors and light emitting units are possible.

In some examples, the device integrates both the light emitting units and the photosensitive sensors in a single tubular channel structure to be inserted into the ear canal.

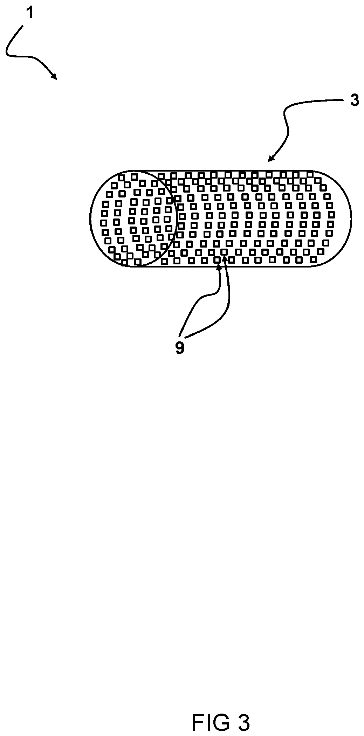

Fig. 3 shows a schematic diagram of a side view of an embodiment of a device 1. The plurality of photosensitive sensors 9 are distributed over the outer portion of the body of the channel structure 3, such that in the expanded position, the photosensitive sensors 9 are distributed over the ear wall of the ear canal. The distributed photosensitive sensors 9 are embedded in a predetermined substantially uniform pattern at the surface of the body, wherein the plurality of photosensitive sensors 9 are disposed in multiple rows and columns on the surface of the body, allowing for the collection of measurement data from various locations on the inner surface of the ear canal. Different patterns and distributions may be employed.

In some examples, a photosensitive sensor 9 may be a broadband detector configured to capture light between the visible spectrum and 1000nm. This allows the selection of wavelengths of the light sources, such as a 600nm red LED and an 850nm near-infrared LED. By comparing the two wavelengths, information about blood oxygen levels can be obtained, as blood reacts differently to these wavelengths. However, also embodiments without a broadband photodetector are envisaged. For instance, alternatively, white light can be used with two types of photodetectors (possibly with filters) that detect specific wavelengths.

In some examples, the device 1 is a radial compliant tubular spO2 sensor. Advantageously, the SpO2 measurements can be significantly less impacted by dynamic conditions. The inner ear canal/tube of the user is suitable for continuous measurements over an extended period of time. Additionally, the location of the PPG is closer to the brain, overcoming a relatively large delay (e.g. approx. 12-second delay) often experienced with finger PPG. Moreover, a channel structure 3 is an open structure, that provides for a PPG system that does not obstruct or hinder the ear entrance, mitigating bacterial growth and reducing the risk of a potential inflammation.

The device 1 may provide for a flexible (e.g. stent structure) PPG for the inner ear or ear canal, with photosensitive sensors 9 (cf. photodetectors) mounted on the flexible channel structure 3 (e.g. on the stent struts). The photosensitive sensors may be positioned on either the inside or outside of the channel structure 3, keeping the ear entrance open and ensuring that the photosensitive sensors are pressed against the ear-canal wall. In some examples, the light emitting units (e.g. LEDs) can be located on the top of the ear shell, creating an illumination path that passes through the skull and part of the brain. Separating the light emitting units and the photosensitive sensors enables to minimize space requirements within the ear canal.

In some examples, a multitude of broadband photosensitive sensors (array or matrix) can be integrated into the stent design channel structure. This array of photosensitive sensors allows for the selection of optimally aligned photodetectors after insertion, eliminating the need for manual repositioning.

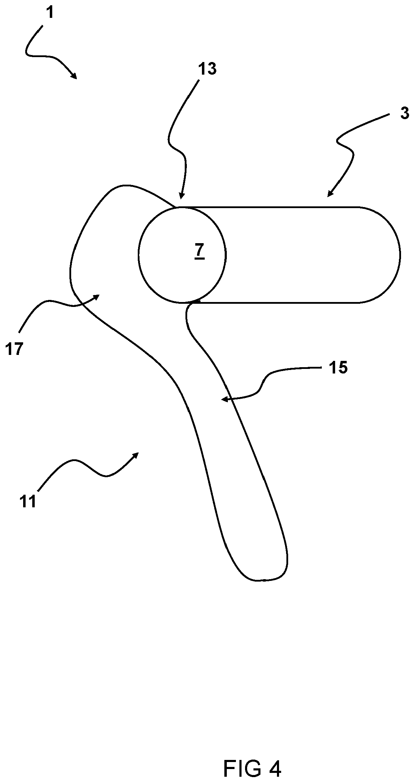

Fig. 4 shows a schematic diagram of an embodiment of a device 1. The channel structure 3 is connected to an outer piece 11, wherein the outer piece 11 extends outwardly from the user's ear when the channel structure 3 is inserted into the ear canal. The outer piece 11 is configured to conform to the shape of the user's ear for providing a secure fit of the outer piece 11 of the device 1 during use.

In this example, the outer piece 11 preserves an open pathway 13 to the open channel 7 of the channel structure 3 such that sound and air can pass through to the open channel 7 of the channel structure 3. The outer piece 11 houses at least a power supply unit and one or more further electronic components of the device.

In some alternative embodiments, it is also possible that the outer piece 11 does not preserve an open pathway, but that rather a microphone is used for providing sound of the environment.

The one or more light emitting units of the device 1 can be arranged on the channel structure 3 and/or at one or more locations of the outer piece 11.

In this example, the outer piece 11 is a rod shaped structure, wherein the rod shaped structure includes a protruding portion 15 extending away from a body 17 connected to the longitudinal channel structure 3.

The outer piece 11 may provide for an unblocked connection to the channel structure 3 in different ways. Advantageously, an open link to the channel structure can be guaranteed.

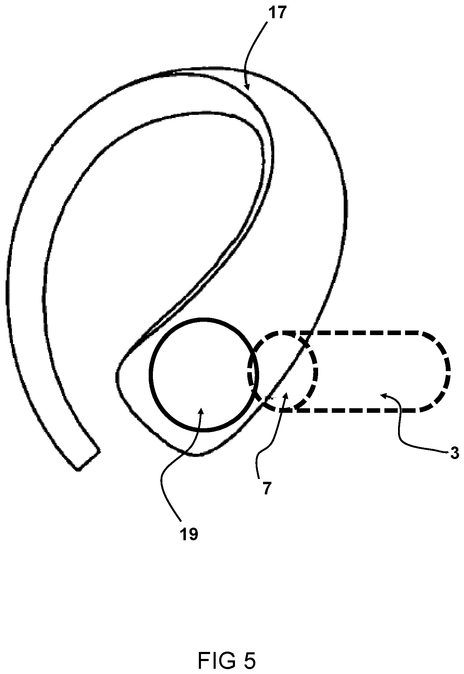

Fig. 5 shows a schematic diagram of an embodiment of a device 1. The outer piece 11 includes a hook-shaped attachment member 17 configured to engage and secure the device 1 to a user's ear by wrapping around the outer contour of the user's ear. One or more light emitting units can be arranged on the hook-shaped attachment member 17. In this example the hook-shaped attachment member 17 preserves an open pathway 19 towards the open channel 7 of the channel structure 3.

The attachment member 17 may foster an unhindered passage with the channel structure 3 in different ways, thereby guaranteeing an access to the channel structure.

In some alternative examples, the outer piece 11 is hook-shaped. The outer piece may thus be configured to contact the skin above the ear when used by the user. In some examples, the outer piece 11 may comprise a plurality of light-emitting units, such as LEDs, the light-emitting units being configured to transmit light through the part of the head towards the ear, wherein the photosensitive sensors of the device are pushed against the interior ear wall.

The outer piece may be connected to the body of the channel structure by means of a flexible connector, the flexible connector allowing for movement and adjustment of the outer piece relative to the body to accommodate different ear and head shapes and sizes. The outer piece may have a contoured surface configured to conform to the shape of the user's head, the contoured surface facilitating optimal contact between the outer piece and the skin above the ear.

The plurality of light-emitting units on the outer piece are configured to emit light at one or more predetermined wavelengths, the predetermined wavelengths being selected for optimal penetration through the head and absorption by blood vessels in the ear wall.

The plurality of light-emitting units on the outer piece and/or attachment member may be arranged in a predetermined pattern and located in a predetermined region, such that an efficient transmission of light from the light-emitting units through the head and towards the ear is facilitated.

A processing or control unit of the device may be configured to selectively activate or deactivate the plurality of light-emitting units on the outer piece and/or attachment member, the control unit allowing for the adjustment of light intensity, wavelength, and/or emission patterns to optimize oxygen level measurements in the blood.

The processing unit of the device may be configured to analyze the received light data from the plurality of photosensitive sensors and the emitted light data from the light-emitting units on the outer piece and/or attachment member to determine the oxygen level in the blood of the surrounding tissue in the ear wall. In some examples, the outer piece and/or attachment member comprises a user interface, the user interface being configured to receive user input for controlling the device, such as turning the device on or off, adjusting settings, or initiating data transmission to an external device among other things.

It will be appreciated that other designs of attachment members may also be used. For example, the attachment member may be configured to fit inside the concha/auricle of the user. In some examples, a concha-adaptable attachment member is provided that can conform to the shape and contours of an individual user's ear concha, wherein the concha-adaptable component comprises a flexible and resilient material capable of adapting to a wide range of ear concha sizes and shapes to provide a secure and comfortable fit. Various other alternative designs are possible.

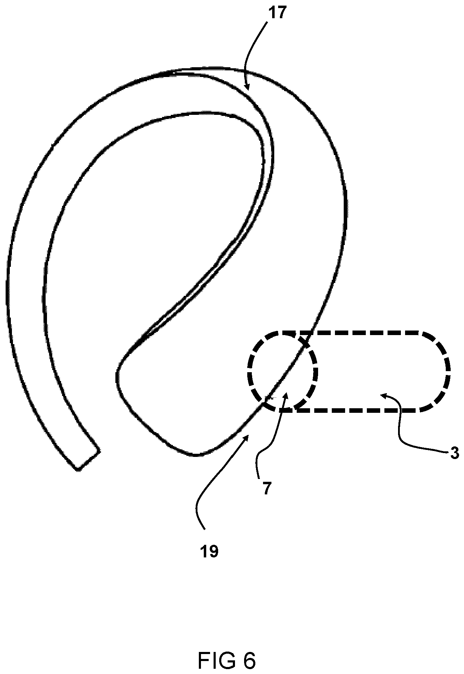

Fig. 6 shows a schematic diagram of an embodiment of a device 1. In this example, the open pathway 19 is preserved in a spacing between the channel structure 3 and the hook-shaped attachment member 17.

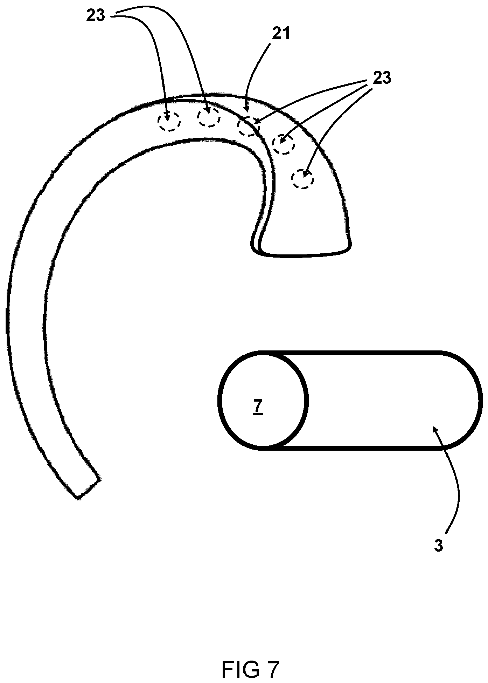

Fig. 7 shows a schematic diagram of an embodiment of a device 1. The device 1 includes a separated unit 21 which is physically detached from the rest of the device 1 encompassing the channel structure 3 and the outer piece. The outer piece can have various shapes and forms and is not illustrated in this figure. The separated unit 21 is configured to be placeable at or in proximity of the ear, and wherein the separated unit includes one or more light emitting units 23. In this example, a plurality of light emitting units 23 are arranged. However, it is also possible to arrange a different number of lights emitting units 23. It is also possible to use only a single light emitting unit. Furthermore, the light emitting units 23 may also be positioned and/or dimensioned differently. The light emitting units 23 may have the same or different characteristics/specifications.

In this example, the device 1 includes a wireless interface between the separated unit 21 and said rest of the device 1, for example to the outer piece 11 connected to the channel structure 3. The wireless interface may be configured to enable data communication over one or more wireless protocols for example for synchronized operation, data transmission, control, etc.

It will be appreciated that, additionally or alternatively, the one or more light emitting units 23 may also be arranged over the outer portion 11 of the body of the channel structure 3, such that in the expanded position, the light emitting units 23 contact the ear wall of the ear canal. The light emitting units may embedded in a predetermined arrangement in the channel structure 3 in different ways.

Arranging the light emitting units 23 outside the ear canal (e.g. approximate or around the ear), can result in a longer travel distance, providing a higher quality signal (pulsating blood).

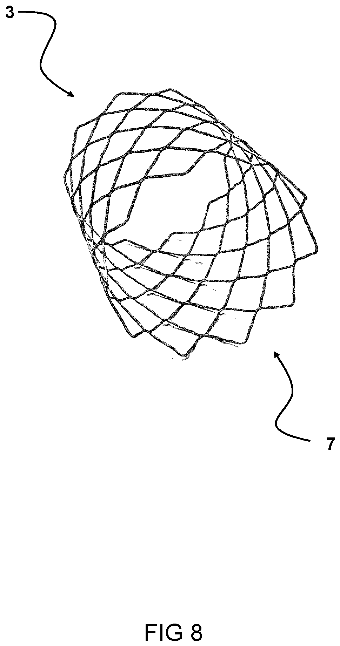

Fig. 8 shows a schematic diagram of a method for a channel structure 3. In this example, the channel structure 3 is configured to be collapsible for insertion into the ear canal and expandable to provide fixation within the ear canal, wherein the channel structure 3 includes a flexible arrangement of mesh-type structures, wherein the channel structure has a first configuration that is radially contracted, and a second configuration that is radially expanded. An arrangement may be provided for adjusting the channel structure from said first configuration towards the second configuration, and/or vice versa. Various stent-like designs may be employed.

The outer portion of the channel structure 3 may be formed by a plurality of intersecting elongate members, wherein the channel structure is configured to be adjustable from a reduced diameter for deployment into the ear canal to an expended diameter, upon application of a radially outward force from the interior of the channel structure, causing deformation of at least a subset of the elongate members to retain the expanded diameter, enabling a securing position of the channel structure within the ear canal.

In some examples, the channel structure 3 of the device 1 is deployed by: inserting the channel structure 3 into the ear cavity in the unexpanded position; applying a stimulus to the channel structure 3 to transition the body from the unexpanded position to the expanded position; and adjusting the position of the channel structure 3 within the ear cavity, if necessary, to ensure that the photosensitive sensors make adequate contact with the ear wall.

The channel structure 3 may have an elongated, cylindrical, and radially-expandable body having a proximal end, a distal end, and a longitudinal axis extending between the proximal end and the distal end. A plurality of interconnected strut members may be arranged forming a lattice structure, said lattice structure configured to push the photosensitive sensors 9 to the ear wall in an expanded state. The strut members may be configured to transition between a collapsed state and an expanded state, wherein in the collapsed state, the channel structure has a reduced diameter to facilitate insertion and delivery to a target site within the ear canal, and in the expanded state, the channel structure has an increased diameter to engage and push the photosensitive sensors and optionally the light emitting units 23 to the ear wall in the ear canal.

The channel structure 3 may have various configurations of a tubular framework. The tubular framework may be configured to be radially compressible for insertion and navigation towards the target location in the ear canal, and radially expandable for deployment and anchoring within the ear canal of the user.

In some examples, the channel structure 3 may include a plurality of helically arranged, interconnected strut elements forming a continuous helical pattern extending along the longitudinal axis of the tubular framework, the helical pattern facilitating radial expansion and contraction while providing circumferential pressure of at least the plurality of photosensitive sensors 9 to the wall of the ear canal.

In some examples, the channel structure 3 includes a plurality of spaced-apart ring segments along the longitudinal axis, each ring segment being formed by a subset of interconnected strut elements, wherein the ring segments and helical pattern provide longitudinal flexibility for adapting to the ear canal.

The stent-like channel structure 3 may be deployed in various ways. In some examples, a balloon-expandable stent deployment is employed, involving mounting a compressed channel structure on a balloon catheter, navigating the catheter to the target site within the ear canal, and then inflating the balloon to expand the channel structure against the wall of the ear canal. Once the channel structure is in place, the balloon is deflated and removed.

In some alternative examples, a self-expanding deployment of the channel structure 3 is employed. The self-expanding channel structure may be compressed and constrained within a delivery sheath. Once the delivery device reaches the target site within the ear canal, the sheath is retracted, allowing the channel structure to expand on its own and conform to the wall of the ear canal. The delivery device is then removed.

In some alternative examples, a smaller stent structure is first deployed within a larger stent-like channel structure 3. The smaller stent is expanded, followed by the expansion of the outer, larger stent-like channel structure.

The ear canal of a human is around 2.5 cm long and 1 cm in diameter with a slight curve. Providing the channel structure 3 with a stent design, such as laser-cut Nitinol stents, which can offer small wall thickness and full compliance (e.g. a 120-micrometer wall thickness), allows the structure to adapt to the ear's curvature in an improved way. The channel structure with stent design can effectively withstand ear canal movement caused by eating, speaking, sporting, etc. Moreover, the channel structure with stent design also offers the advantage of easy insertion and removal using existing deployment technology.

It will be appreciated that although a tubular design of the channel structure 3 is shown in the figures, various other designs are also envisaged, such as but not limited to a C-shaped structure, a spiral structure, a closed or open tube structure, a flexible foam structure, or a combination of such structures. In some examples, the channel structure 3 comprises a flexible material, the flexible material facilitating the expansion of the outer portion 5 of the body of the channel structure 3.

Various embodiments may be implemented using hardware elements, software elements, or a combination of both. Examples of hardware elements may include processors, microprocessors, circuits, application specific integrated circuits (ASIC), programmable logic devices (PLD), digital signal processors (DSP), field programmable gate array (FPGA), logic gates, registers, semiconductor device, microchips, chip sets, et cetera. Examples of software may include software components, programs, applications, computer programs, application programs, system programs, machine programs, operating system software, mobile apps, middleware, firmware, software modules, routines, subroutines, functions, computer implemented methods, procedures, software interfaces, application program interfaces (API), methods, instruction sets, computing code, computer code, et cetera.

Herein, the invention is described with reference to specific examples of embodiments of the invention. It will, however, be evident that various modifications, variations, alternatives and changes may be made therein, without departing from the essence of the invention. For the purpose of clarity and a concise description features are described herein as part of the same or separate embodiments, however, alternative embodiments having combinations of all or some of the features described in these separate embodiments are also envisaged and understood to fall within the framework of the invention as outlined by the claims. The specifications, figures and examples are, accordingly, to be regarded in an illustrative sense rather than in a restrictive sense. The invention is intended to embrace all alternatives, modifications and variations which fall within the scope of the appended claims. Further, many of the elements that are described are functional entities that may be implemented as discrete or distributed components or in conjunction with other components, in any suitable combination and location.

In the claims, any reference signs placed between parentheses shall not be construed as limiting the claim. The word 'comprising' does not exclude the presence of other features or steps than those listed in a claim. Furthermore, the words 'a' and 'an' shall not be construed as limited to 'only one', but instead are used to mean `at least one', and do not exclude a plurality. The term "and/or" includes any and all combinations of one or more of the associated listed items. The mere fact that certain measures are recited in mutually different claims does not indicate that a combination of these measures cannot be used to an advantage.

## Claims

1. A device for performing oxygen saturation measurements, the device comprising a longitudinal channel structure removably insertable into an ear canal, the channel structure having an outer portion configured to expand into an expanded position, wherein in the expanded position the channel structure is configured such that its outer portion exerts pressure against an inner surface of the ear canal of the user, wherein a longitudinal open channel is formed by a body of the channel structure to maintain an opening in the ear canal when the channel structure is placed in its expanded position in the ear canal, wherein the channel structure includes a plurality of photosensitive sensors which are utilized for performing pulse oximetry measurements when the device is in the expanded position.

2. The device according to claim 1, wherein the channel structure is a predominantly elongated open channel structure, wherein the open channel of the channel structure has a cross-sectional area ratio, defined as the open channel cross-sectional area divided by the total cross-sectional area of the channel structure, that is higher than 50%, more preferably higher than 60%, even more preferably higher than 70%.

3. The device according to claim 1 or 2, wherein the channel structure is connected to an outer piece, wherein the outer piece extends outwardly from the user's ear when the channel structure is inserted into the ear canal, wherein the outer piece is configured to conform to the shape of the user's ear for providing a secure fit of the outer piece of the device during use, wherein the outer piece preserves an open pathway to the channel structure, and wherein the outer piece houses at least a power supply unit and one or more further electronic components of the device.

4. The device according to any one of the preceding claims, wherein the device includes one or more light emitting units configured to transmit light at one or more wavelengths, wherein the plurality of photosensitive sensors on the channel structure are configured to receive the emitted light passing surrounding blood vessels and/or bodily tissue of the user so as to measure oxygen saturation levels in the blood of the user, wherein the one or more light emitting units are arranged on the channel structure and/or at one or more locations of the outer piece.

5. The device according to claim 3 or 4, wherein the outer piece is a rod shaped structure, wherein the rod shaped structure includes a protruding portion extending away from a body connected to the longitudinal channel structure.

6. The device according to claim 3, 4, or 5, wherein the outer piece includes a hook-shaped attachment member configured to engage and secure the device to a user's ear by wrapping around the outer contour of the user's ear, wherein one or more light emitting units are arranged on the hook-shaped attachment member.

7. The device according to any one of the preceding claims, wherein the device further includes a separated unit which is physically detached from the rest of the device encompassing the channel structure and the outer piece, wherein the separated unit is configured to be placeable at or in proximity of the ear, and wherein the separated unit includes one or more light emitting units, and wherein the device includes a wireless interface between the separated unit and said rest of the device, wherein the wireless interface is configured to enable data communication over one or more wireless protocols for synchronized operation.

8. The device according to any one of the preceding claims, wherein the plurality of photosensitive sensors are distributed over the outer portion of the body, such that in the expanded position, the sensors are distributed over the ear wall of the ear canal, wherein the distributed photosensitive sensors are embedded in a predetermined substantially uniform pattern on the surface of the body, wherein the plurality of photosensitive sensors are disposed in multiple rows and columns on the surface of the body, allowing for the collection of measurement data from various locations on the inner surface of the ear canal.

9. The device according to any one of the preceding claims, wherein the device includes a processing unit, wherein the processing unit is configured to selectively use data from the plurality of photosensitive sensors based on the received light data such that only sensors with adequate data contribute to the oxygen measurement.

10. The device according to any one of the preceding claims, the channel structure is configured to be collapsible for insertion into the ear canal and expandable to provide fixation within the ear canal, wherein the channel structure includes a flexible arrangement of a mesh-type structure, wherein the channel structure has a first configuration that is radially contracted, and a second configuration that is radially expanded.

11. The device according to any one of the preceding claims, wherein the outer portion of the channel structure is formed by a plurality of intersecting elongate members, wherein the channel structure is configured to be adjustable from a reduced diameter for deployment into the ear canal to an expended diameter, upon application of a radially outward force from the interior of the channel structure, causing deformation of at least a subset of the elongate members to retain the expanded diameter, enabling a securing position of the channel structure within the ear canal.

12. The device according to any one of the preceding claims, wherein the channel structure comprises a flexible material, the flexible material facilitating the expansion of the outer portion of the body.

13. The device according to any one of the preceding claims, wherein the channel structure is a C-shaped structure, a spiral structure, a closed or open tube structure, a flexible foam structure, or a combination of such structures.

14. A method of using a device for performing oxygen saturation measurements, the device comprising a longitudinal channel structure removably insertable into an ear canal, the channel structure having an outer portion configured to expand in an expanded position, wherein in the expanded position the channel structure is configured such that its outer portion exerts pressure against an inner surface of the ear canal of the user, wherein a longitudinal open channel is formed by a body of the channel structure to maintain an opening in the ear canal when the channel structure is placed in its expanded position in the ear canal, wherein the channel structure includes a plurality of photosensitive sensors which are utilized for performing pulse oximetry measurements when the device is in the expanded position, wherein the method includes the steps of:
positioning the channel structure into an ear canal of a user;
applying a stimulus to the channel structure in order to transition the channel structure from an unexpanded position to an expanded position such that its outer portion engages the inner surface of the ear canal.

15. The method according to claim 14, wherein the stimulus applied to the device comprises applying a mechanical force/pressure, releasing a radially compression pressure on the outer portion of the channel structure, activating a movement mechanism, changing a temperature or applying an electrical current for actuation.
